(19) Europäisches Patentamt
European Patent Office
Office européen des brevets

(11) **EP 2 922 577 B1**

(12) **EUROPEAN PATENT SPECIFICATION**

(45) Date of publication and mention
of the grant of the patent:
**28.08.2019 Bulletin 2019/35**

(21) Application number: **13818529.3**

(22) Date of filing: **19.11.2013**

(51) Int Cl.:
*A61K 49/18* (2006.01)   *A61K 33/26* (2006.01)
*B82Y 5/00* (2011.01)   *B82Y 30/00* (2011.01)
*C01G 49/08* (2006.01)   *A61K 49/00* (2006.01)
*B82Y 15/00* (2011.01)   *C01G 49/02* (2006.01)

(86) International application number:
**PCT/PL2013/050031**

(87) International publication number:
**WO 2014/081322 (30.05.2014 Gazette 2014/22)**

(54) **SUPERPARAMAGNETIC IRON OXIDE NANOPARTICLES WITH ULTRA-THIN POLYMER LAYERS**

SUPERPARAMAGNETISCHE EISENOXIDNANOPARTIKEL MIT ULTRADÜNNEN POLYMERSCHICHTEN

NANOPARTICULES D'OXYDE DE FER SUPERPARAMAGNÉTIQUE COMPORTANT DES COUCHES POLYMÈRES ULTRAMINCES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2012 PL 40170612**

(43) Date of publication of application:
**30.09.2015 Bulletin 2015/40**

(73) Proprietor: **Uniwersytet Jagiellonski**
**31007 Krakow (PL)**

(72) Inventors:
• **ZAPOTOCZNY, Szczepan**
**30-381 Kraków (PL)**
• **KANIA, Gabriela**
**34-300 Zywiec (PL)**
• **SZPAK, Agnieszka**
**30-864 Kraków (PL)**
• **NOWAKOWSKA, Maria**
**30-433 Kraków (PL)**

(56) References cited:
**WO-A1-99/62079**        **US-A- 5 262 176**
**US-A1- 2004 146 855**

• **HERNANDEZ R ET AL: "Influence of iron oxide nanoparticles on the rheological properties of hybrid chitosan ferrogels", JOURNAL OF COLLOID AND INTERFACE SCIENCE, ACADEMIC PRESS, NEW YORK, NY, US, vol. 339, no. 1, 1 November 2009 (2009-11-01), pages 53-59, XP026542277, ISSN: 0021-9797, DOI: 10.1016/J.JCIS.2009.07.066 [retrieved on 2009-08-06]**

**Description**

FIELD OF INVETNION

The invention relates to superparamagnetic iron oxide nanoparticles (SPION) with ultra-thin polymer layers made of superparamagnetic cores of iron oxides, co-precipitated with polycation and hydrophilic coatings composed of ultra-thin layers of polyelectrolytes, increasing the biocompatibility and relaxivity of the entire structure in aqueous media. The invention relates also to, described above, iron oxide nanoparticles, which hydrophilic coating has been functionalized with a fluorescent probe, gadolinium compound, antibodies, aptamers of antibodies or polyelectrolyte layer with fluorinated groups.

STATE OF THE ART

The contrast agents commonly used in magnetic resonance imaging (MRI) consist of gadolinium ions complexes (Geraldes 2009), which make the contrast agents toxic and require prompt removal from the body after the examination. The use of these substances can also lead to development of diseases such as nephrogenic systemic fibrosis, especially for patients suffering from acute renal failure. Therefore, other substances that act as contrast agents and are also safe for use in humans and animals, are sought. Superparamagnetic nanoparticles according to the invention provide an alternative to currently used contrast agents. The magnetic properties of these particles allow reducing the minimum dose that is introduced into the patient in order to obtain a similar imaging effect as that obtained in the case of currently used paramagnetic substances.

There are many methods of synthesis of superparamagnetic iron oxide particles (Tartaj 2003, Teja 2009, Laurent 2008, Tartaj 2005), however for biological applications, co-precipitation method from aqueous solutions of iron salts is usually used. The advantages of the method are: simplicity of preparation, high efficiency and easy procedures of the material isolation, then its further modification or transferring into the aqueous phase. A limitation of the available methods is, often occurring, aggregation of obtained nanoparticles. In order to stabilize the resulting structures, the surface of the particles is coated with, inter alia, polymeric or low molecular weight stabilizers (Laurent 2008, Gupta 2005). In addition, the assimilation of superparamagnetic iron oxide nanoparticles by the organism or the immune reactions caused by them depends mainly on the nanoparticles size. The particles larger than 200 nm are captured by the spleen, and then removed by the phagocytic cells. However, superparamagnetic iron oxide nanoparticles with diameters of less than 10 nm are rapidly removed from the body by the kidneys. These phenomena result in a shortening of the circulation time of nanoparticles in the blood stream.

To overcome the abovementioned obstacles, in the present invention the superparamagnetic iron oxide nanoparticles with particle size from 10 nm to 200 nm are administered intravenously. Nanoparticles of this size are characterized by a maximum time of circulation in the blood stream. They are small enough to be disregarded by the phagocytic cells, resulting in their deeper penetration, even to small capillaries that allows achieving an effective distribution of superparamagnetic iron oxide nanoparticles in target tissues. Thus, for biomedical applications, it is important to obtain particles with small size and low polydispersity. In addition, contrast media should be biocompatible, preferably biodegradable and selective, i.e., they should be characterized by an ability to bind selectively to specific tissues/cells of the body. Covering of the nanoparticles with suitable materials (coating) increases their biocompatibility, but also allows for the adequate chemical modifications, the connection with selected antibodies etc. to provide the selective placement of the nanoparticles in specific tissues of the body (Laurent 2008).

The contrast agents containing ultrasmall superparamagnetic iron oxide nanoparticles and superparamagnetic iron oxide nanoparticles (for example ferumoxides, FeREX or ferumoxtran-10) coated mainly by dextran or its derivatives are known (Geraldes 2009, Laurent 2008). Dextran is a natural, biocompatible polysaccharide having a relatively high molecular weight, usually containing a certain content of branched chain. However, the use of dextran in case of available contrast agents has some disadvantages. In the process of coating many nanoparticles are embedded together inside a single polymer structure that leads to the formation of even micrometer-sized systems with high polydispersity. These particles are more readily taken up by macrophages in the body, what reduces the circulation time of the contrast agents in the blood stream. Furthermore, a large ratio of the polymer weight to the weight of the nanoparticles results in a decreasing of the contrast magnetic properties, making it necessary to increase the effective dose administered to the patient before preparing the diagnostic tests with MRI technique. The binding of the nanoparticles with a polymer layer is often weak. In the case of dextran, the dilution leads to the polymer desorption from the surface of the nanoparticles, resulting in their aggregation and easier capturing by the immune system (Laurent 2008). Moreover, dextran coatings are also disadvantageous because of the antidextran antibodies present in the body. Therefore, administration of the dextran-containing contrast agent can induce cytotoxicity (Yuan 2008).

In order to eliminate abovementioned barriers, in present invention dextran is proposed to be replaced by another polysaccharide, namely, chitosan and its derivatives. Chitosan is a non-toxic, biocompatible and biodegradable polymer. It is obtained in the process of deacetylation of chitin, which is found in shells of crabs, shrimp or squid. Chitosan macromolecules include, similarly to dextran, -OH groups, but also -NH$_2$ groups, which significantly extend the ability of chitosan modification. Unfortunately, chitosan is soluble only in an acidic medium in which the protonation of amino groups occurs. This can be a significant limitation in the use of non-modified chitosan, for example, under conditions of neutral pH (pH of human blood = 7.4). Chitosan has already been used in the delivery of insulin (Agnihotri 2004). It is also used as a component of bandages and other dressings. Another advantage of chitosan in comparison to other natural polysaccharides is its ability to chelate metal ions, resulting in the formation of stable metal-to-polymer bonding (Yuan 2008). Therefore, it can be successfully used for stabilization of the superparamagnetic iron oxide nanoparticles and may be a starting point for further selective modification of the coated nanoparticles.

Polymers, such as dextran and chitosan, are in the group of hydrophilic polymers. Therefore, they constitute a water-permeable material, which, in the context of the present invention, represents a significant advantage. Indeed, the covering of superparamagnetic cores with ultra-thin layers of the chitosan derivatives or heparin, influences on the penetration of water molecules through the coating closer to the cores. In addition, the diffusion of these molecules is slowed down by the coating (Kwak 2003), therefore the contact of the water molecules with superparamagnetic core is longer, and this translates into an improvement in the magnetic properties due to increased transverse relaxivity $r_2$.

International patent application WO 99/62079 discloses a method for the preparation of magnetic nanoparticles by producing a nuclei made of polymers which are metal chelating agents, and growing a magnetic metal oxide coating thereon. The obtained material is composed of magnetic nanoparticles consisted of the polymeric metal chelating agent core, which can be e.g. removed by burning off, and the magnetic metal oxide coating. The used polymers may be polysaccharides that should contain appropriate metal chelating groups. Importantly, the magnetic coating is prepared on the preformed polymer nuclei by contacting them with appropriate metal salts in a sequential process.

In the state of the art, it is know the method of preparation of ferrogels with magnetite nanoparticles embedded in chitosan hydrogels. Such ferrogel is formed by addition of alkali coagulant solution to an acidic solution of unmodified chitosan with an appropriate iron salt that leads to simultaneous precipitation of the nanoparticles and gelation of chitosan (Hernandez, 2009).

The patent application US2004/146855 A1 describes the method of preparation of superparamagnetic particles in a polysaccharide matrix crosslinked either chemically or physically. In the first step, Fe(II) ions have to diffuse into a polysaccharide matrix or create a complex with, e.g., chitosan. In the next step, the obtained material is oxidized by some nitrate (NO$^{3-}$) at higher temperature (70-80 °C).

The patent application US5262176 A describes the method of preparation of superparamagnetic iron oxide nanoparticles covered by an unmodified non-ionic polysaccharides (only dextran is reported) in a cold gelation process. The proposed method does not provide surface charge to the nanoparticles disabling further coating and functionalization of them using electrostatically-driven processes that is claimed in the present invention. What is more, as reported in this patent application the dextran coating is susceptible to heat stress leading to dissociation of dextran from the surface of nanoparticles due to relatively weak interactions that is not the case in the present invention where much stronger electrostatic forces act.

DETAILED DESCRIPTION OF THE INVENTION

This invention relates to superparamagnetic iron oxide nanoparticles with ultrathin polymer layers characterized in that they consist of superparamagnetic iron oxide cores co-precipitated with polycation, and a hydrophilic coating consisting of at least one ultrathin layer of a polyelectrolyte.

Preferably, the first ultrathin layer of a polyelectrolyte obtained by co-precipitation of the nanoparticles in the presence of the polymer and subsequent odd layers, deposited on the surface of the obtained nanoparticles, consist of a polycation. Particularly preferably, when the polycation is selected from the group consisting of natural or modified polysaccharides containing positively charged groups, such as ammonium, pyridinium or phosphonium and most preferably, when the polycation is chitosan modified with ammonium groups.

Also preferably, the second ultra-thin layer of the polyelectrolyte and more even layers, are composed of a polyanion. Particularly preferably, when the polyanion is a polymer from the group consisting of natural or modified polysaccharides containing negatively charged functional groups such as sulfonate, sulfate, carboxyl or phosphate, including most preferably, when the polyanion is a polymer constituting an anionic chitosan derivatives or heparine, particularly carboxymethylchitosan modified with sulfur trioxide complex or heparin.

Preferably, the nanoparticles are suspended in the aqueous phase. Also preferably, the nanoparticles are coated with such a number of coatings that their average size is 5-200 nm and/or diameter of iron oxide core is 5-20 nm and/or the thickness of the polymer coating is 0.1-100 nm.

An advantage of the invention is the increasing of biocompatibility and relaxivity of the whole nanoparticle structure in an aqueous medium associated with the occurrence of coating consisting of ultrathin layers of polyelectrolytes. The polycation layer consisting of chitosan modified with ammonium groups gives the nanoparticles a durable positive charge, and the imposing of another layer of polyelectrolyte gives the nanoparticles a durable negative charge. It influences, inter alia, favorable on the stability of suspensions of nanoparticles in water.

In a preferred embodiment, the superparamagnetic nanoparticles are characterized in that the hydrophilic coating is functionalized by attaching to it a fluorescent probe and/or a gadolinium compound and/or antibodies and/or aptamers of antibodies and/or polyelectrolyte layers having fluorinated groups.

Particularly preferably, the fluorescent probe is selected from the group consisting of: fluorescamine, fluorescein, cyanine, rhodamines, or functionalized quantum dots.

Also preferably, the gadolinium compound is selected from the group consisting of chelate of gadolinium ions with diethylenetriaminepentaacetic acid (DTPA) or ethylenediaminetetraacetic acid (EDTA).

Also preferably, the antibody is selected from the group consisting of monoclonal antibodies against cell adhesion molecules, including, in particular, VCAM-1, against P-selectin or E-selectin.

Also preferably, the aptamers of antibodies are selected from the group consisting of aptamers of monoclonal antibodies against cell adhesion molecules or against P-selectin or E-selectin.

Also preferably, the polyelectrolyte layers having fluorinated groups are poly(N,N-dimethyl-N-4'-vinylbenzyl-N-2-(perfuorooctanoyl-N'-methylimino)ethylammonium chloride)-co-(N,N,N-trimethyl-N-4'-vinylbenzylammonium chloride) (AK-St-F).

The advantage of superparamagnetic iron oxide nanoparticles of the invention, which are coated with a cationically modified chitosan and, in next step, carboxymethylchitosan anionically modified with sulfur trioxide, is the small size, low size polydispersity, durable surface charge, stability in water, and much better magnetic properties (relaxivity) than available in the prior art contrast agents.

The resulting suspension of nanoparticles can find a number of biomedical applications. The abovementioned advantages of nanoparticles allows them to be used as the contrast agent in smaller doses (lower concentration of iron) than that obtained using other available contrast agents while providing the same effect on MRI. Introduction of gadolinium on the SPION surface allows obtaining images not only T2-weighted, due to the presence of iron oxide in the structure, but also the T1-weighted, which greatly enhances the contrast between collected scans. Gadolinium in the form of $Gd^{3+}$ cation complexes with DTPA or EDTA is introduced on the surface of the nanoparticles by addition of these chelating groups to the both, the positively and negatively charged polymer coating. Whereas, the coating made of polyelectrolyte layer having fluorinated groups allows using such modified nanopartcicles as contrast agents for simultaneous imaging $^1$H NMR and $^{19}$F NMR, which, due to its high fluorine nuclei response in this kind of research, also makes it easier to track changes in generated images. The introduction of fluorine atoms on the surface of the nanoparticles can be carried out using both, the cationic and the anionic polymer, i.e., regardless of the sign of the surface charge of the modified nanoparticles. The covering with a layer of heparin allows, inter alia, better differentiate imaging of atherosclerotic endothelium. Joining to the SPION surface of a specific antibody or specific aptamer of antibody, results in specific binding of the nanoparticles with the selected tissue/cell of the human body, including, for example, an inflamed blood vessel wall, which also improves the quality of obtained MRI images. While, the introduction of a fluorescent probe to the surface of SPION enables greater use of nanoparticles in medical diagnostics, since such functionalized nanoparticles will be used as a diagnostic agent used simultaneously in magnetic resonance imaging as well as in fluorescence microscopy. The suspensions of superparamagnetic iron oxide nanoparticles with surface appropriately modified can be used also for the cellular labeling or separation of cells, tissue repair, drug delivery, hyperthermia or magnetofection.

A BRIEF DESCRIPTION OF THE DRAWINGS

The invention is described in more detail embodiments in the accompanying drawings, in which:

Fig. 1          shows the macromolecular structure of the ammonium derivative of chitosan ;
Fig. 2 & 3      show TEM images of nanoparticles of example 2;
Fig. 4          shows TEM images of nanoparticles of example 6;
Fig. 5 & 6      show TEM images of nanoparticles of example 5;
Fig. 7 & 8      show graphs illustrating the distribution of the core diameter of superparamagnetic iron oxide nanoparticles;
Fig. 9          shows a graph of the hydrodynamic diameter of the colloidal nanoparticles of examples 2, 6 and 7;
Fig. 10         shows an example of photograph illustrating the crystal planes obtained using HREM for nanoparticles of example 2;
Fig. 11         shows IR spectra of ammonium derivative of chitosan and nanoparticles of example 2 and 5;
Fig. 12         shows images from magnetic force microscope for colloids of example 2;
Fig. 13         shows a graph of the magnetic susceptibility of nanoparticles of example 2 as a function of temperature;
Fig. 14         shows a graph of the magnetization (M) as a function of the magnetic induction ($\mu_0$H) for colloid of

example 2;

Fig. 15, 16 & 17    represent specimens showing the interaction of colloids of example 2 and 6 with human blood;

Fig. 18, 19 & 20    represent thermograms recorded for cationically modified chitosan and nanoparticles of examples 2 and 5;

Fig. 21    shows the excitation and emission spectra of the iron oxide nanoparticles with fluorescamine attached to their surface;

Fig. 22    shows MRI maps originating from *in vivo* observation of impact of iron oxide nanoparticles of example 5 and 6 on liver $T_2$ relaxation time maps.

## EMBODIMENTS OF THE INVENTION

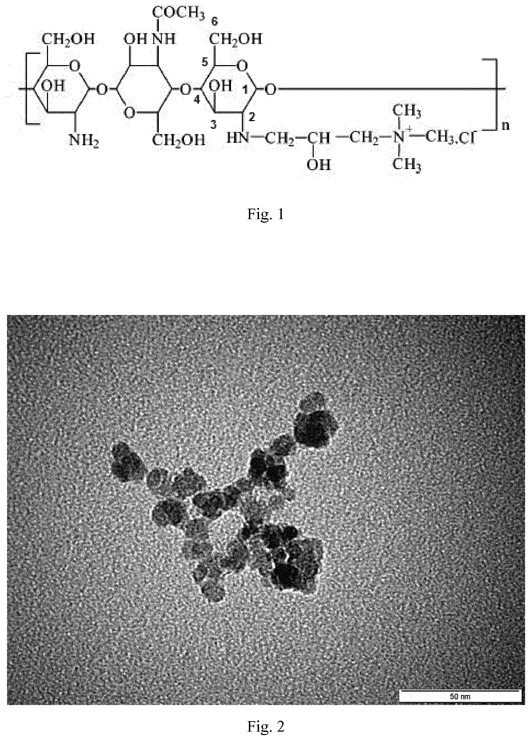

Superparamagnetic iron oxide nanoparticles according to the invention were prepared by one-step method of synthesis, consisting of co-precipitating of iron(II) and iron(III) ions in presence of biocompatible cationic polymer, cationically modified chitosan. Cationically modified chitosan synthesis was carried out according to the method described in the literature (Cho 2006), resulting in formation of polymer with structure presented in Fig. 1. In examples 1-5 cationically modified chitosan having a degree of substitution of 95%, defined on the basis of nuclear magnetic resonance spectra, was used (Bulwan 2009).

*Example 1. Synthesis of superparamagnetic iron oxide nanoparticles coated with cationically modified chitosan (CCh)*

0.1622 g $FeCl_3 \cdot 6H_2O$ and 0.0596 g $FeCl_2 \cdot 4H_2O$ (concentrations molar ratio of Fe(III):Fe(II) = 2:1) were added to 50 ml CCh solution with concentration 1g/l in 0.1M NaCl, at pH 1.5. The system was closed, purged with argon for 10 min, sonicated (sonicator type SONIC-6 POLSONIC) using continued sonication without cooling of the system. 5 ml 5M $NH_{3(aq)}$ was added to the system and the mixture was sonicated for further 30 min under the same conditions, purging with argon continuously. pH of the mixture after the synthesis was about 10. Continuous sonication of the system led to an increase in the bath temperature to about 40 °C at the end of the process. The resulting suspension was filtered through a syringe filter with pores of 0.2 $\mu$m. For purification of the suspension of coated iron oxide nanoparticles from the residue of reactants magnetic chromatography was applied. 5 ml of suspension was introduced on a column packed with steel wool (ferromagnetic), to which neodymium magnets were applied. On the column filling, the nanoparticles were deposited, then they were washed with 3 ml of deionized water. After removal of the magnets, the column was washed with deionized water, and the purified nanoparticles were collected.

*Example 2. Synthesis of superparamagnetic iron oxide nanoparticles coated with cationically modified chitosan using pulsed sonication with cooling.*

The procedure was as in example 1, except that the pulsed sonication (pulse length of 1 s at 5 s) and the ice-cooling (the temperature constant in the course of about 20 °C) in an ultrasonic bath were applied.

*Example 3. Synthesis of superparamagnetic iron oxide nanoparticles coated with cationically modified chitosan using continuous sonication with cooling.*

The procedure was as in example 1, except that the continuous sonication was applied and the ice-cooling as in example 2 was used.

*Example 4. Synthesis of superparamagnetic iron oxide nanoparticles coated with cationically modified chitosan with the use of mechanical stirrer.*

The procedure was as in example 1, except that the mixing system using a mechanical stirrer (mechanical stirrer EUROSTAR powercontrol-visc PI, IKA-WERKE) was applied instead of sonication. Mixing was performed at the speed of 300 rpm.

*Example 5. Synthesis of superparamagnetic iron oxide nanoparticles coated with cationically modified chitosan with the use of pulse sonication with cooling and with higher concentration of polymer.*

The procedure was as in example 2, except that the CCh solution with concentration of 3g/l in 0.1M NaCl was used.

*Example 6. Covering of nanoparticles coated with cationically modified chitosan using the carboxymethylchitosan anionically modified with sulfur trioxide (ACh).*

5 ml of purified suspension of superparamagnetic iron oxide nanoparticles coated with cationically modified chitosan of example 2 was sonicated for 5 min. 20 ml of ACh solution with concentration of 1g/l in 0.1M NaCl was added and sonication was continued for 10 min. The suspension of nanoparticles coated with ACh was purified from excess of polymer and salt using magnetic chromatography as in example 1.

*Example 7. Coating of nanoparticles with negative surface charge obtained as in example 6 with cationically modified chitosan.*

4 ml of the purified suspension of superparamagnetic iron oxide nanoparticles coated with CCh and then ACh of example 6 was sonicated for 5 min. Then 10 ml of CCh solution having a substitution degree of 56% and a concentration of 1g/l in 0.1 M NaCl was added and sonication was continued for 10 min. The suspension of nanoparticles coated with CCh/ACh/CCh was purified from excess of polymer and salt using magnetic chromatography as in example 1.

*Example 8. Coating of cationically modified chitosan nanoparticles with the use of heparin.*

10 ml of suspension of example 5 (for preparation of the nanoparticles, CCh solution having a substitution degree of 56% was used) was sonicated for 5 min, using continuous sonication. Then, while continuing sonication, 10 ml of a heparin solution having a concentration of 3g/l in 0.2 M NaCl was added. The thus-formed mixture was sonicated for 10 min, proceeding continuous sonication. The suspension of nanoparticles coated with heparin was purified from excess of polymer and salt using magnetic chromatography as in example 1.

*Example 9. Coating of nanoparticles obtained according to example 6, having negative surface chargé, with the use of poly(N,N-dimethyl-N-4'-vinylbenzyl-N2-(perfluorooctanoyl-N'-methylimino)ethylammonium chloride)-co-(N,N,N-trimethyl-N-4'-vinylbenzylammonium chloride) (AK-St-F).*

10 ml of suspension obtained according to example 6 was sonicated for 5 min using continuous sonification. Then, while continuing sonication, 10 ml of AK-St-F solution having a concentration of 3 g/l in 0.2 M NaCl was added. The thus-formed mixture was sonicated for 10 min, proceeding continuous sonication. The suspension of nanoparticles coated with AK-St-F was purified from excess of polymer and salt using magnetic chromatography as in example 1.

*Example 10. Determination of iron in the suspension of nanoparticles.*

In order to determine the concentration of iron in colloids obtained according to the procedures described in examples 1-5, spectrophotometric method based on the formation of colored complexes of iron(II) with phenanthroline was used. 1 ml of 1M HCl was added to 1 ml of tested sample, heating in hot water to dissolve the iron oxide nanoparticles. Then, in order to reduce Fe(III) to Fe(II), vitamin C was added in a 1:1 molar ratio concerning the theoretical iron content of the sample. In the next stage, the solution was diluted 50-fold and 6.25 ml of it was pipetted into a 25 ml flask, to which 3 ml of 0.2% solution of 1,10-phenanthroline hydrate in 0.08M HCl was also added, whereafter filled up to the 25 ml line with deionized water. After 10 min the absorbance for the solution was measured at wavelength $\lambda_{max}$ = 512 nm. The iron contents in the obtained solutions (examples 1-5) were calculated from the measured values of absorbance, based on the previously determined calibration curve for various concentrations of Fe(II). These results, together with the yield of the superparamagnetic iron oxide nanoparticles synthesis are shown in Table 1.

**Table 1**

| Colloid from the example | cFe exp [mg/ml] | cFe theor [mg/ml] | Synthesis yield [%] |
|---|---|---|---|
| 1 | 0.189 | 0.920 | 20.5 |
| 2 | 0.220 | 0.915 | 24.0 |
| 3 | 0.019 | 0.918 | 2.1 |
| 4 | 0.159 | 0.916 | 17.,4 |
| 5 | 0.365 | 0.915 | 39.9 |

*Example 11. Examination of the stability of colloids in deionized water.*

The colloid samples were sonicated for 5 min before performing the zeta potential measurements. The measurements were performed using the instrument Zetasizer Nano ZS from Malvern. The results of zeta potential measurements for colloids of examples 1-7 are summarized in Table 2.

**Table 2**

| Colloid from the example | Zeta potential [mV] |
| --- | --- |
| 1 | 51.2 |
| 2 | 47.3 |
| 3 | 47.0 |
| 4 | 49.3 |
| 5 | 41.8 |
| 6 | -40.6 |
| 7 | 46.7 |

Colloidal solutions are stable when the values of the zeta potentials of the particles dispersed phase are less than -30 mV or greater than 30 mV. The data collected in Table 2 suggest that, the stable colloids were obtained as a result of particle synthesis according to the procedures described in examples 1-7. The colloidal particles obtained in example 6 have a negative zeta potential, which indicates that they have been covered with an anionic polymer resulting in changing of the surface charge from positive to negative. However, the colloidal particles of example 7 have a positive zeta potential, indicating complete surface covering by cationic polymer resulting in obtaining and the surface charge reversal from negative to positive. This experiment proves that the method for easy, controlled manipulation of charge on the surface of superparamagnetic iron oxide nanoparticles by adsorption of oppositely charged polyelectrolytes, has been developed.

*Example 12. Nanoparticles core size determination using TEM.*

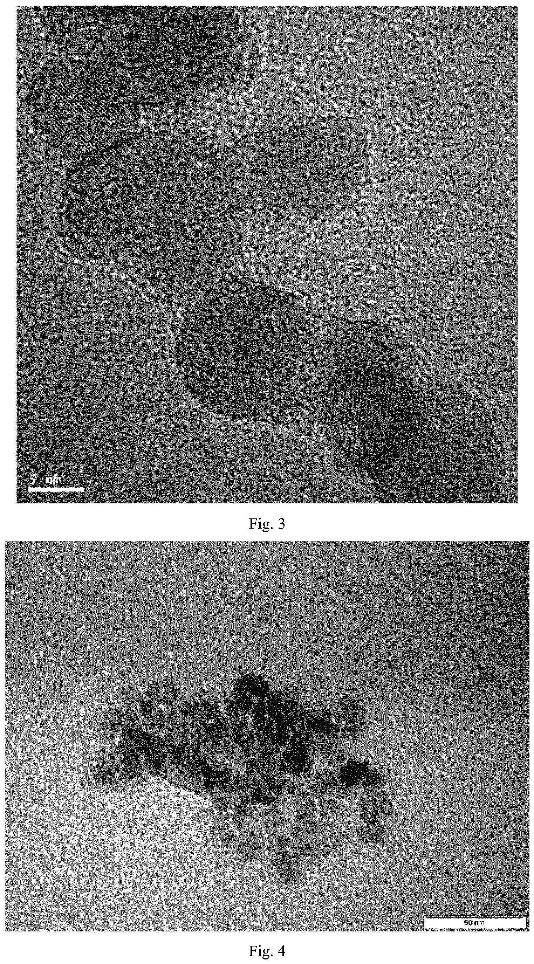

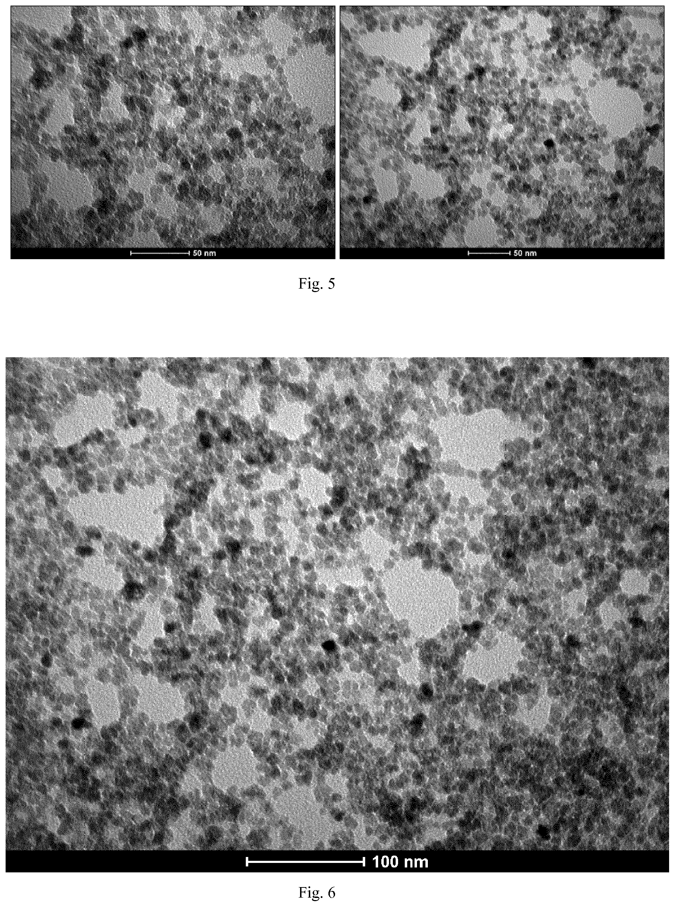

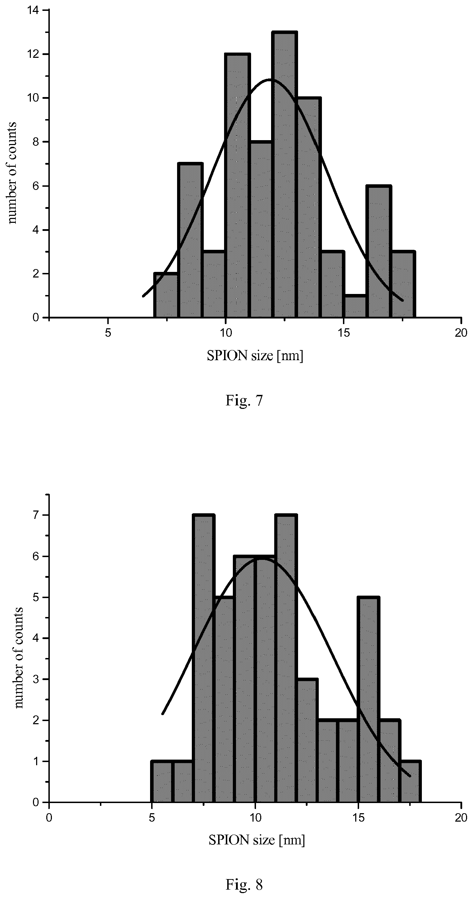

In order to determine the size of nanoparticles, the colloids obtained according to the procedures described in examples 2 and 6 were sonicated for 2 min. Then, small volumes of these solutions were applied to the carbon grid and were allowed to dry under cover. However, in order to imaging the nanoparticles synthesized according to the procedure described in example 5 (for the synthesis of the colloid sample based on example 5, CCh solution having a degree of substitution determined from nuclear magnetic resonance spectra and equal 56 %) the colloid was lyophilized, and then dried nanoparticles were dissolved in methanol and sonicated for 2 min. The so-obtained preparation was applied to a carbon grid and left under cover for solvent evaporation. The measurement was performed using Tecnai G2 F20 (200kV) transmission electron microscope equipped with a field emission gun FEG in bright-field and high-resolution mode (HREM). The images of colloid of example 2 are shown in Fig. 2 and 3, colloid of example 6 is illustrated in Fig.4, while nanoparticles of example 5 are shown in Fig. 5 and 6. The diameter of the nanoparticles of example 2 was determined on the basis on images captured in high-resolution mode, while the size of superparamagnetic iron oxide nanoparticles of example 6 was determined using TEM, bright-field mode. In each case about 100 counts of the size of nanoparticles were performed. Graphs showing the diameter distribution of superparamagnetic iron oxide nanoparticles are shown in Fig. 7 and 8. The individual figures show:

- bright-field TEM for nanoparticles of example 2 in Fig.2,
- HREM for nanoparticles of example 2 in Fig.3
- bright-field TEM for nanoparticles of example 6 in Fig. 4,
- 50 nm scale bright-field TEM for nanoparticles of example 5 in Fig. 5,
- 100 nm scale bright-field TEM for nanoparticles of example 5 in Fig. 6,
- size distribution for nanoparticles of example 2 in Fig. 7,
- size distribution for nanoparticles of example 6 in Fig. 8.

Nanoparticles of example 2 have a size of 11.9 $\pm$ 1.7 nm, while superparamagnetic iron oxide nanoparticles of example 6 are characterized by size 10.1 $\pm$ 2.9 nm. Significant measurement error in the case of nanoparticles of example 6 occurs due to the fact, that the counts were made from images taken in bright field (less accurate) because this type of samples does not allow obtaining good quality of images at high resolution. As it can be seen in Fig. 5 and 6, the increasing amount of the polymer during the co-precipitation reduces the size of formed nanoparticles; in the images the objects with dimensions smaller than 10 nm can be seen. Aggregates shown in Fig. 2-6 were formed during the aggregation process as a result of measurements procedure, which forces the drying of the samples.

*Example 13. Determination of the nanoparticles hydrodynamic diameters.*

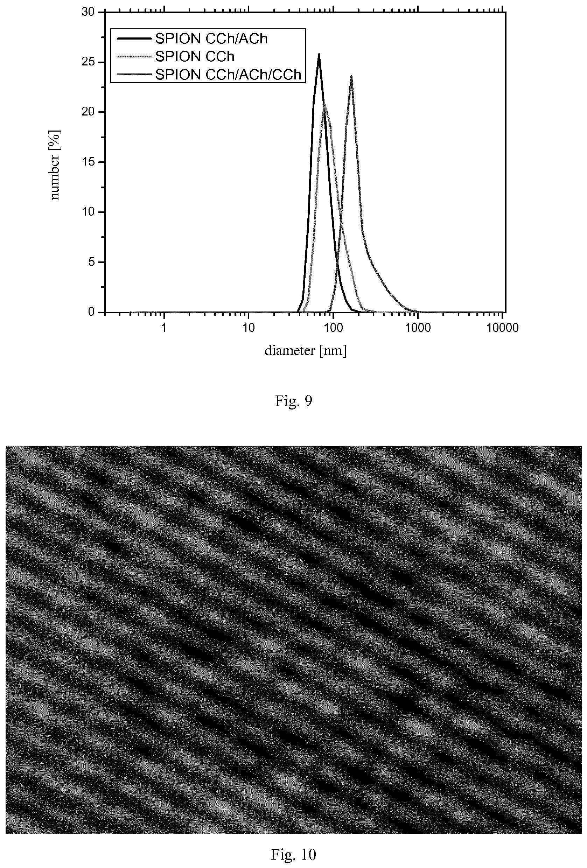

In order to determine the nanoparticles hydrodynamic diameters, colloids of examples 2, 6 and 7 were sonicated for 5 min. The measurements were carried out using Zetasizer Nano ZS provided by Malvern, by using dynamic light scattering (DLS) measured at 173° in temperature 25°C. The obtained results are presented in Fig. 9. The distributions of nanoparticles hydrodynamic diameters in an aqueous suspension show that nanoparticles of example 2 oscillate around 80 nm, SPION of example 6 around 70 nm and SPION of example 7 - 160 nm. In view of the results of example 10 it can be concluded that one nanoparticle is formed of a number of cores made up of iron oxide of about 10-12 nm in diameter connected to the polymer chain.

*Example 14. Determination of the crystal structure of nanoparticles.*

The crystallographic structure of iron oxide forming superparamagnetic iron oxide nanoparticles due to the syntheses described in Examples 1-6 was determined based on images of the nanoparticles of example 2 captured in high-resolution mode. A sample image showing the crystallographic planes obtained by using HREM for colloid of example 2 is presented in Fig. 10. The determination of the interplanar crystal spacings was also made on the basis of Fourier transform of obtained HREM images for example 2. Table 3 gives obtained interplanar spacings along with associated to them spacings for the sample of example 2.

**Table 3**

| Measured interplanar crystal spacing d [Å] | Interplanar crystal spacing d for $Fe_3O_4$* [Å] | Miller indices for planes |
|---|---|---|
| 4.82 | 4.85 | 111 |
| 3.06 | 2.97 | 220 |
| 2.58 | 2.53 | 311 |
| 2.80 | 2.81 | 221 |
| 1.68 | 1.71 | 422 |
| 1.55 | 1.56 | 423 |
| *\* On the basis of the calculations resulting from the unit cell parameters for a = 8.396 Å* | | |

Planes (220) for $Fe_3O_4$ are presented in Fig. 10 (Iidaa 2007). Spacings between the crystallographic planes are equal 0.306 nm. The data presented in Table 3 show that co-precipitation of iron(II) and iron(III) with cationically modified chitosan does not change the crystal structure of the obtained $Fe_3O_4$.

*Example 15. Determination of the interaction nature between the polymer and the surface of nanoparticles.*

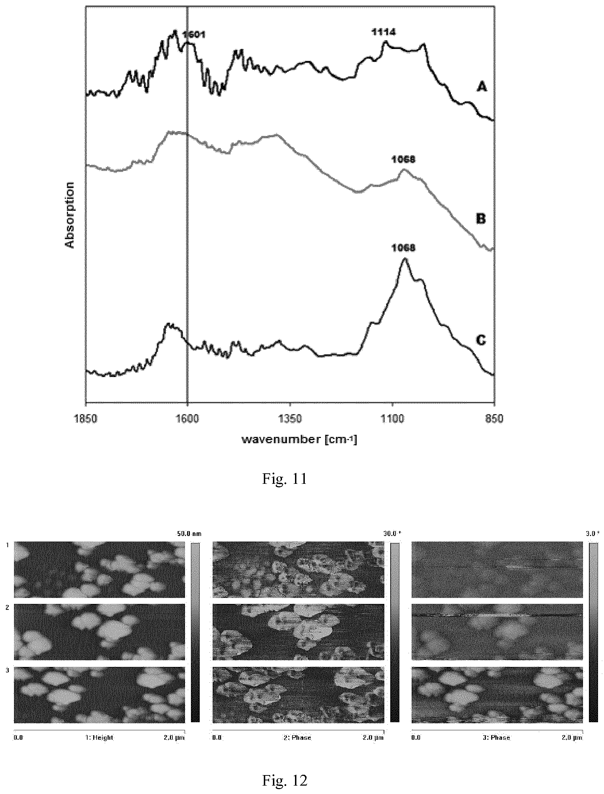

In order to determine how the polymer is binding to iron oxide, infrared spectra for the samples of examples 2 and 5 were performed. The glass plate with a reflective coating (MirrIR glass provided by Kevley Technologies) was washed with ethanol, and then 20 drops of the suspension of nanoparticles of examples 2 and 5 were deposited on it (in different places) by waiting after each drop deposition for solvent to evaporate. IR spectra of the nanoparticles, which are shown in Fig. 11 (line B for nanoparticles of example 5 and line C for nanoparticles of example 2), were collected in transreflection mode using spectrometer FT-IR Varian 670. IR spectrum of pure cationically modified chitosan (Fig. 11, line A) was collected by using of the KBr pellet and spectrometer with Fourier transform EQUINOX 55 provided by Bruker. The spectra in Fig. 11 shows that the band at 1601 cm$^{-1}$, attributed to vibrations of primary amino group, disappears for nanoparticles of examples 2 and 5. This indicates that iron ions from the nanoparticles are chelated by the amino groups of the polymer (Wang 2008). There is also a shift of C-O stretching vibration bands at 1114 cm$^{-1}$ (CCh) up to 1068 cm$^{-1}$ (examples 2 i 5), showing that there is an interaction between hydroxyl group at $C_3$ and magnetite nanoparticles (Hernández 2009).

*Example 16. Imaging the magnetic properties of nanoparticles.*

In order to imaging the magnetic properties of superparamagnetic iron oxide nanoparticles, the measurements with the used of magnetic force microscopy were performed. For this purpose, a silicon wafer was cut into tiles, which were provided in a solution that is a mixture of conc. $H_2SO_4$ and 30% $H_2O_2$ at a volume ratio of 1:1 for 30 min to clean them. Plates were rinsed with large amounts of distilled water and dried in a stream of argon. The colloid of example 2 was sonicated for 5 min and then diluted 10-fold. 10 $\mu$l of the prepared sample were loaded on the aforementioned plate, allowed to stand for 15 min, and then dried in a stream of argon. The measurement was performed using atomic force microscope equipped with a controller Nanoscope IVA (Bruker, USA), using silicon tip coated with Co/Cr magnetic layer (Bruker). The measurement was made in the oscillatory mode (called "tappingmode") using the so-called "lift mode" in imaging the magnetic properties. The resulting images from the magnetic force microscope for colloid of example 2 are shown in Fig. 12 (from left to right: topography, phase imaging, imaging magnetic forces). The magnetic measurements were carried out for different distances between the tip and the tested sample (1 - 75 nm, 2 - 50 nm, 3 - 25 nm).

Fig. 12 shows that the colloid of example 2 exhibits magnetic properties. This is evidenced by changes in contrast in the pictures on the right side resulting from the reduction of the distance between the tip and the sample (interaction distance effect). Magnetic interactions are long-range interactions, and therefore are observed even at high tip-to-sample distances (1 in Figure 12, on the right side). Aggregates observed in the pictures are the result of the action of capillary forces during the plate drying.

*Example 17. Measurement of the magnetic susceptibility of nanoparticles.*

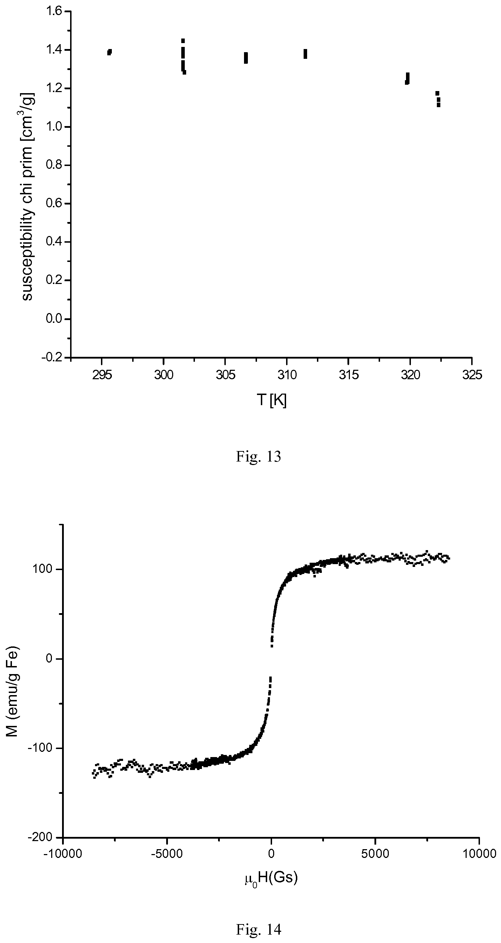

To explore the magnetic susceptibility, the colloid from example 2 was placed in the container Lake ShoreCryotronics Inc. with symbol 700 SC-10 and tested. The real component of the magnetic susceptibility as a function of the AC magnetic field was measured at a frequency of 189 Hz using Stanford SR 830 nanovoltmeter. Magnetic susceptibility as a function of temperature is shown in Fig. 13 as a plot of the real component of the magnetic susceptibility as a function of temperature for the colloid of example 2.

A value of magnetic susceptibility illustrated in Fig. 13 is approximately constant and equals 1.4 $cm^3$/g. Positive susceptibility value indicates that the nanoparticles are made of paramagnetic material.

*Example 18. The measurements of nanoparticles magnetization.*

In order to determine the dependence of magnetization (M) on the magnetic field induction ($\mu_0 H$), colloid of example 2 was placed in the container Lake ShoreCryotronics Inc. with symbol 700 SC-10 and tested. In measurements the controller of the vibration magnetometer Lake Shore Model 7300 was used. The sample was kept at 200 K using Janis flow cryostat. The plot of above relation is shown in Fig. 14 (dependency of magnetization on the magnetic field induction for colloid of example 2).

Fig. 14 shows a typical relationship $M=f(\mu_0 H)$ for superparamagnetic substance. The linear relationship is not observed (paramagnetic materials) or there is no hysteresis characteristic for ferromagnetic substances.

The resulting value of the saturation of magnetization 123 $\pm$ 12 emu/g Fe is close to the value measured for magnetite (Jung 1995), and taking into account the total weight of the coated nanoparticles, that value equals 61 $\pm$ 6 emu/g, what is comparable to values measured for similar superparamagnetic nanoparticles (Iidaa 2007).

*Example 19. Determination of relaxivity for the colloidal solution of nanoparticles.*

Measurements were carried out for, properly diluted with deionised water, samples of colloids of examples 2, 5 and 6 and FEREX, the commercial contrast agent. The measurements of time relaxation $T_1$ and $T_2$ were carried out using MR 4,7 T experimental magnetic resonance tomograph with horizontal superconducting magnet provided by Bruker. In order to obtain the relaxivity $r_1$ and $r_2$ for individual colloids the following equations were used:

$$R_1 = \frac{1}{T_1} \quad R_2 = \frac{1}{T_2}$$

$$R_1 = R_1^0 + r_1 \cdot c_{Fe} \quad R_2 = R_2^0 + r_2 \cdot c_{Fe}$$

where:

$R_1$ ($R_2$) - longitudinal (transverse) relaxation rate;
$T_1$ ($T_2$) - longitudinal (transverse) relaxation time;
$R_1^0$ ($R_2^0$) - longitudinal (transverse) relaxation rate of the sample without a contrast agent;
$r_1$ ($r_2$) - longitudinal (transverse) relaxivity;

$c_{Fe}$ - concentration of iron in the tested contrast agent.

The results are presented in Table 4 showing the relaxivities of colloids of examples 2, 5 and 6 and Ferex.

**Table 4.**

| | $c_{Fe}[\mu g/ml]$ | $T_1$ [ms] | $T_2$ [ms] | $R_1$ [1/s] | $R_2$ [1/s] | $r_1[s^{-1}(\mu g/ml)^{-1}]$ | $r_2[s^{-1}(\mu g/ml)^{-1}]$ |
|---|---|---|---|---|---|---|---|
| deionized water | 0 | 3945.535 | 3367.175 | 0.253 | 0.297 | | |
| Colloid of example 2 | 2.20 | 2905.722 | 106.471 | 0.344 | 9.392 | $0.036 \pm 0.008$ | $4.1 \pm 0.7$ |
| | 1.10 | 3355.022 | 336.797 | 0.298 | 2.969 | | |
| | 0.22 | 3476.840 | 1071.545 | 0.288 | 0.933 | | |
| Colloid of example 5 | 3.65 | 2676.982 | 116.599 | 0.374 | 8.576 | $0.031 \pm 0.003$ | $2.3 \pm 0.2$ |
| | 1.82 | 3135.589 | 279.195 | 0.319 | 3.582 | | |
| | 0.36 | 3530.280 | 1385.030 | 0.283 | 0.722 | | |
| Colloid of example 6 | 3.02 | 2932.147 | 49.522 | 0.341 | 20.193 | $0.028 \pm 0.003$ | $6.60 \pm 0.05$ |
| | 0.60 | 3564.371 | 241.697 | 0.281 | 4.137 | | |
| FeREX | 2.53 | 2948.600 | 150.322 | 0.339 | 6.652 | $0.035 \pm 0.003$ | $2.5 \pm 0.3$ |
| | 1.26 | 3277.343 | 236.700 | 0.305 | 4.225 | | |
| | 0.25 | 3818.088 | 898.820 | 0.262 | 1.113 | | |

The suitability of the obtained nanoparticles as contrast agents in MRI measurements can be inferred based on the relaxivity values $r_1$ and $r_2$. The higher the value of $r_2$ for the colloid, with the same value $r_1$ for commercial FeREX, the greater the contrast enhancement (darkening field) in magnetic resonance imaging. The colloids obtained in examples 2 and 6 demonstrate, therefore, much better magnetic properties than the tested commercial contrast agent.

*Example 20. Influence of nanoparticles on coagulation of erythrocytes.*

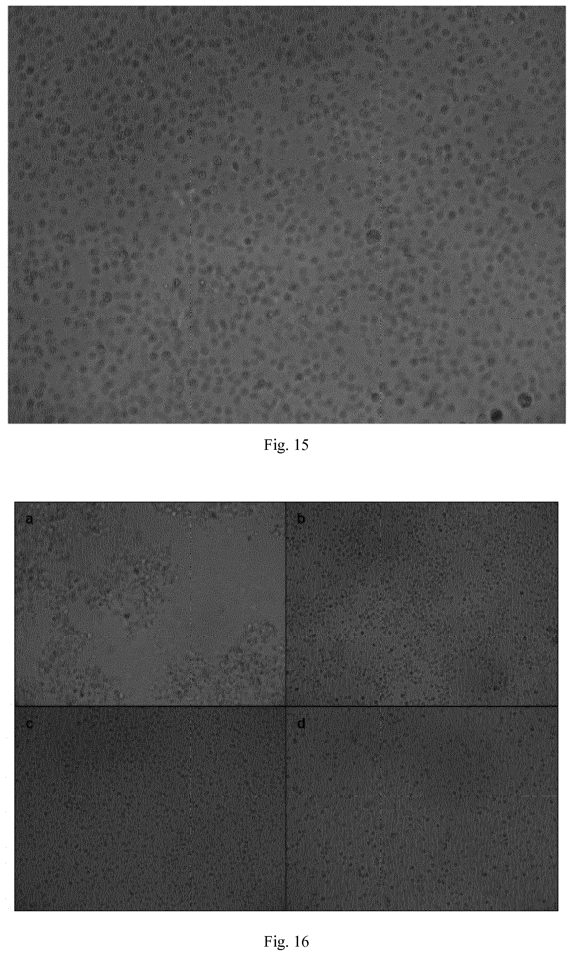

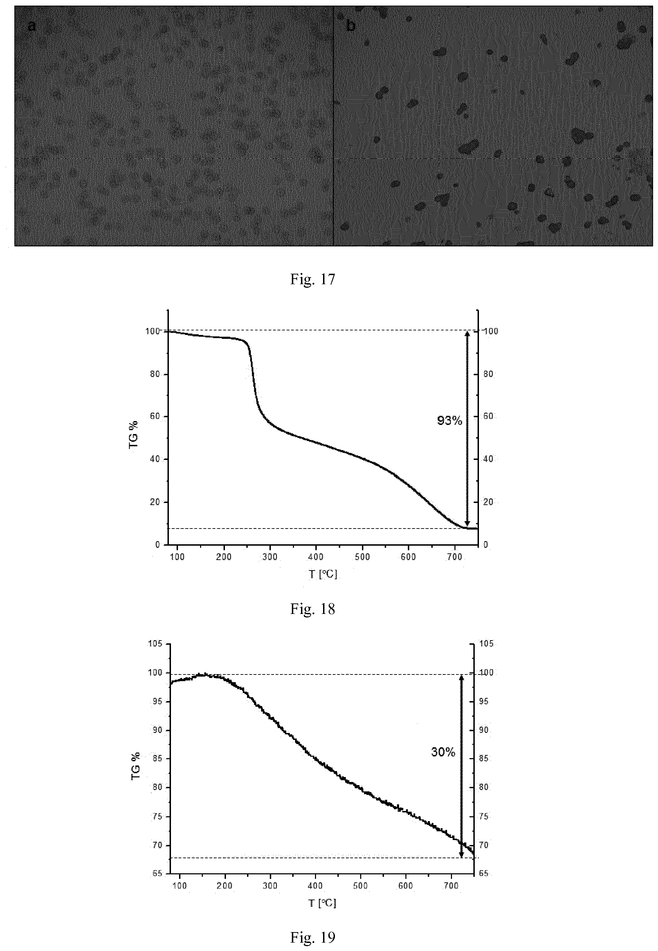

In order to examine the impact of colloids of examples 2 and 6 on human blood, 4 $\mu$l of blood and 18 $\mu$l of corresponding colloid were spotted on microscope slides. After mixing, the specimens were covered with a cover glass and observed under ECLIPSE LV100D optical microscope provided by Nikon. The resulting images are shown in Fig.15-17. Fig. 17 shows human blood (a) and aggregates formed from erythrocytes (b), as models for the studied colloids. Fig. 15 shows the colloid of example 6 with the blood, and Fig.16 shows the colloid of example 2 (a) without dilution, (b) 10-fold diluted, (c) 100-fold diluted, (d) 1000-fold diluted with blood.

The possible biological use of colloids of examples 2 and 6 can be concluded on the basis of these findings. The colloid of example 2 has to be diluted before administration to prevent erythrocyte aggregation. However, the colloid of example 6 can be used even in concentrated doses, because the aggregation of erythrocytes is not observed. Carboxymethylchitosan anionically modified with sulfur trioxide, well attached to the surface of nanoparticles with a positive charge (superparamagnetic iron oxide nanoparticles coated with cationically modified chitosan), provides reversal of the surface charge to negative, and thus greater biocompatibility with human blood.

*Example 21. Determination of the polymer content in the nanoparticles.*

In order to determine the CCh content in co-precipitated iron oxide nanoparticles, the colloid samples of examples 2 and 5 (for the synthesis of the colloid sample of example 5, CCh with 56% degree of substitution was used) were lyophilized. Thus obtained powders were placed in platinum crucible, and these in thermogravimetric analyzer Netzsch STA 449 F1 Jupiter. The measurement was carried out at temperatures 23-810°C with rate of increase 10°C/min in argon atmosphere. Thermograms recorded for the pure cationically modified chitosan as well as for nanoparticles of examples 2 and 5 are shown in Fig.18, 19, 20:

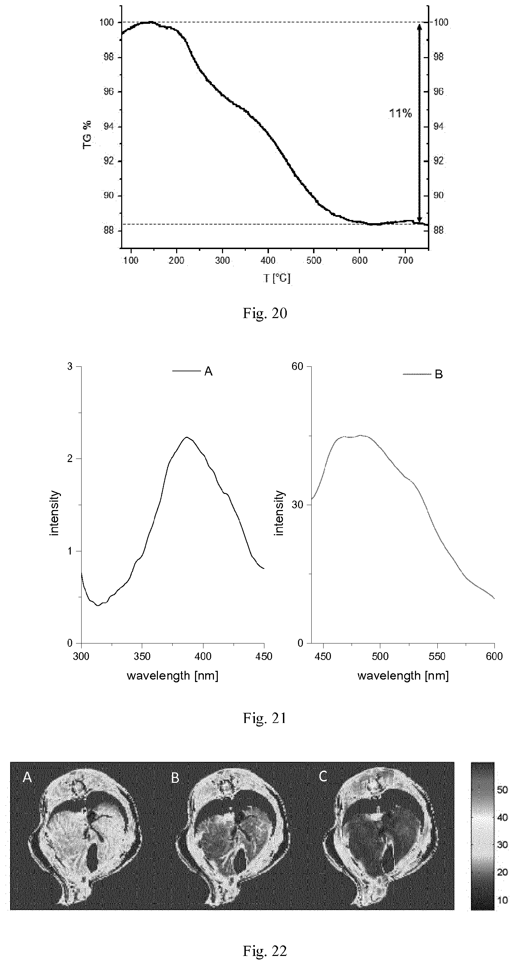

- Thermogram for pure CCh in Fig. 18,
- Thermogram for nanoparticles of example 2 in Fig. 19,
- Thermogram for nanoparticles of example 5 in Fig. 20.

The thermogram in Fig. 18 shows that under the tested conditions, the weight loss for pure CCh is 93%. For the sample of example 2, the weight loss, associated with CCh decomposition, equals 30% (Fig. 19), while for the sample of example 5 is 11% (Fig. 20). On this basis, the CCh content in the colloid samples of examples 2 and 5 were determined, giving the following values:

- nanoparticles of example 2 contain 32% of CCh (0.3/0.93=0.32),
- nanoparticles of example 5 contain 12% of CCh.

Although in the synthesis of superparamagnetic iron oxide nanoparticles with CCh of example 5, a solution with higher concentration of CCh was used, its content in the system is less than when using a solution with lower concentration. It should be noted, however, that in this particular case, for the colloid of example 5, a polymer with a different degree of substitution of ammonium groups was used, which have a significant effect on the properties of the final product.

*Example 22. Iron oxide nanoparticles with the fluorescent probe attached to their surface.*

10 ml of suspension of example 5 (for the nanoparticles synthesis, CCh with 56% degree of substitution was used) was mixed with 2 ml of fluorescamine solution in acetone at a concentration of 1mg/ml. After 15 min, the acetone was removed by purging of the mixture with an inert gas. Then the nanoparticles were purified by magnetic chromatography. In order to confirm the functionalization of positively charged surface of SPION with fluorescent probe which is fluorescamine, the purified nanoparticles suspension was properly diluted with deionized water. Then an excitation ($\lambda_{em}$ = 500 nm; Fig. 21A) and emission spectra ($\lambda_{ex}$ = 395 nm; Fig. 21B) were recorded using LS 55 spectrofluorometer provided by PerkinElmer. In Fig. 21 the characteristic bands of the product resulting from the reaction of fluorescamine with primary amino groups presented in CCh structure are shown, which confirms the obtainment of SPION with positive surface charge functionalized with fluorescent probe.

*Example 23. In vivo observation of the effect of the iron oxide nanoparticles of example 5 (degree of substitution 56%) and 6 based on the example of liver relaxation time $T_2$ maps.*

The measurement A (Fig. 22) was carried out after putting the BALB/c mouse in the scanner. Then nanoparticles of example 6 were administrated in a dose of 0.55 mg/kg in 5% glucose solution, and measurement B (Fig. 22) was carried out. After about 12 min, the nanoparticles of example 5 were administrated in a dose of 0.55 mg/kg in 5% glucose solution, and measurement C (Fig. 22) was carried out. The nanoparticles were administered through a catheter inserted into the femoral artery. MSME sequence was used in the measurements, TE = 4 ms, TR = 1500 ms, effective TR is about 2000 ms - every second breath, double gating, FOV = 22x22 mm, MTX 128x128.

As it can be noticed, each following map of $T_2$ relaxation time shows $T_2$ at lower value. It can therefore be concluded, that iron oxide nanoparticles obtained according to the examples 5 and 6 behave in the *in vivo* systems as contrast agents shortening the $T_2$ relaxation time.

CITED LITERATURE:

1. Agnihotri, S.A., Mallikarjuna, N.N., Aminabhavi, T.M. (2004). Journal of Controlled Release, 100, 5-28.
2. Bulwan, M., Zapotoczny, S., Nowakowska, M. (2009). Soft Matter, 5, 4726-4732.
3. Cho, J., Grant, J., Piquette-Miller, M., Allen, C. (2006). Biomacromolecules, 7, 2845-2855.
4. Geraldes, C.F.G.C. S., Laurent, C.F.G.C. (2009). Contrast Media Mol. Imaging, 4, 1-23.
5. Gupta, A.K., Gupta, M. (2005). Biomaterials, 26, 3995-4021.
6. Hernández, R., Zamora-Mora, V., Sipaja-Ballestero, M., Vega-Baudrit, J., Lopez, D., Mijangos, C. (2009). Journal of Colloid and Interface Science, 339, 53-59.
7. Iidaa, H., Takayanagia, K., Nakanishib, T., Osakaa, T. (2007). Journal of Colloid and Interface Science, 314, 274-280.
8. Jung, C.W., Jacobs, P. (1995). Magnetic Resonance Imaging, 13, 661-674.
9. Kwak, S., Lafleur, M. (2003). Macromolecules, 36, 3189-3195.
10. Laurent, S., Forge, D., Port, M., Roch, A., Robic, C., Vander Elst, L., Muller, R.N. (2008). Chemical Reviews, 108 , 2064-2110.
11. Tartaj, P., del Puerto Morales, M., Veintemillas-Verdaguer, S., González-Carreño, T., Serna, C.J. (2003). Journal of Physics D: Applied Physics, 36, R182-R197.
12. Tartaj, P., Morales, M.P., González-Carreño, T., Veintemillas-Verdaguer, S., Serna, C.J. (2005). Journal of Magnetism and Magnetic Materials, 290-29, 28-34.

13. Teja, A.S., Pei-Yoong Koh (2009). Progress in Crystal Growth and Characterization of Materials, 55, 22-45.

14. Wang, Y., Li, B., Zhou, Y., Jia, D. (2008). Polymers for Advanced Technologies, 19, 1256-1261.

15. Yuan, Q., Venkatasubramanian, R., Hein, S., Misra, R.D.K. (2008). ActaBiomaterialia, 4, 1024-1037.

2855.

4. Geraldes, C.F.G.C. S., Laurent, C.F.G.C. (2009). Contrast Media Mol. Imaging, 4, 1-23.

5. Gupta, A.K., Gupta, M. (2005). Biomaterials, 26, 3995-4021.

6. Hernández, R., Zamora-Mora, V., Sipaja-Ballestero, M., Vega-Baudrit, J., López, D., Mijangos, C. (2009). Journal of Colloid and Interface Science, 339, 53-59.

7. Iidaa, H., Takayanagia, K., Nakanishib, T., Osakaa, T. (2007). Journal of Colloid and Interface Science, 314, 274-280.

8. Jung, C.W., Jacobs, P. (1995). Magnetic Resonance Imaging, 13, 661-674.

9. Kwak, S., Lafleur, M. (2003). Macromolecules, 36, 3189-3195.

10. Laurent, S., Forge, D., Port, M., Roch, A., Robic, C., Vander Elst, L., Muller, R.N. (2008). Chemical Reviews, 108 , 2064-2110.

11. Tartaj, P., del Puerto Morales, M., Veintemillas-Verdaguer, S., González-Carreño, T., Serna, C.J. (2003). Journal of Physics D: Applied Physics, 36, R182-R197.

12. Tartaj, P., Morales, M.P., González-Carreño, T., Veintemillas-Verdaguer, S., Serna, C.J. (2005). Journal of Magnetism and Magnetic Materials, 290-29, 28-34.

13. Teja, A.S., Pei-Yoong Koh (2009). Progress in Crystal Growth and Characterization of Materials, 55, 22-45.

14. Wang, Y., Li, B., Zhou, Y., Jia, D. (2008).Polymers for Advanced Technologies, 19, 1256-1261.

15. Yuan, Q., Venkatasubramanian, R., Hein, S., Misra, R.D.K. (2008). ActaBiomaterialia, 4, 1024-1037.

## Claims

1. Superparamagnetic iron oxide nanoparticles with ultra-thin polymer layers, **wherein** nanoparticles consist of:

   a. superparamagnetic iron oxides cores co-precipitated with a polycation,
   b. hydrophilic layer made of at least one ultrathin layer of a polyelectrolyte.

2. Superparamagnetic iron oxide nanoparticles according to claim 1, **wherein** first ultra-thin layer of polyelectrolyte is obtained as a result of co-precipitation of nanoparticles in the presence of polymer, and other odd layers, deposited on the surface of the obtained nanoparticles, consist of a polycation.

3. Superparamagnetic iron oxide nanoparticles according to claim 2, **wherein** polycation is selected from the group of natural or modified polysaccharides containing positively charged groups, such as: ammonium, pyridinium or phosphonium.

4. Superparamagnetic iron oxide nanoparticles according to claims 2 or 3, **wherein** the polycation is chitosan modified with cationic ammonium groups.

5. Superparamagnetic iron oxide nanoparticles according to claim 1, **wherein** second ultra-thin layer of polyelectrolyte and more even layers consist of a polyanion.

6. Superparamagnetic iron oxide nanoparticles according to claim 5, **wherein** the polyanion is a polymer from the group consisting of natural or modified polysaccharides containing negatively charged functional groups, such as sulfonate, sulfate, carboxyl or phosphate.

7. Superparamagnetic iron oxide nanoparticles according to claims 5 or 6, **wherein** the polyanion is a polymer constituting the anionic chitosan derivative or heparin.

8. Superparamagnetic iron oxide nanoparticles according to the claim 7, **wherein** the polymer forming an anionic chitosan derivative is carboxymethylchitosan modified with sulfur trioxide complex.

9. Superparamagnetic iron oxide nanoparticles according to the claim 1, **wherein** the nanoparticles form a suspension in aqueous phase.

10. Superparamagnetic iron oxide nanoparticles according to claim 1, **wherein** the nanoparticles are coated with such a number of coatings that their average size is 5-200 nm and/or the diameter of the iron oxide core is 5-20 nm and/or the thickness of the polymer coating is 0.1-100 nm.

11. Superparamagnetic iron oxide nanoparticles according to claim 1, **wherein** hydrophilic coating is functionalized by attaching a fluorescent probe and/or a gadolinium compound and/or antibodies and/or antibodies aptamers and/or polyelectrolyte layer having fluorinated groups to it.

12. Superparamagnetic iron oxide nanoparticles according to claim 11, **wherein** the fluorescent probe is selected from the group consisting of: fluorescamine, fluorescein, cyanine, rhodamines or functionalized quantum dots.

13. Superparamagnetic iron oxide nanoparticles according to claim 11, **wherein** the gadolinium compound is selected from the group consisting of chelates of gadolinium ions with diethylenetriaminepentaacetic acid (DTPA) or ethylenediaminetetraacetic acid (EDTA).

14. Superparamagnetic iron oxide nanoparticles according to claim 11, **wherein** the antibody is selected from the group consisting of monoclonal antibodies against cell adhesion molecules, including, in particular, VCAM-1, monoclonal antibodies against P-selectin or monoclonal antibodies against E-selectin.

15. Superparamagnetic iron oxide nanoparticles according to claim 11, **wherein** the antibody aptamers are selected from the group consisting of monoclonal antibody aptamers against cell adhesion molecules, against P-selectin or E-selectin.

16. Superparamagnetic iron oxide nanoparticles according to claim 11, **wherein** the polyelectrolyte layers having fluorinated groups are poly(N,N-dimethyl-N-4'-vinylbenzyl-N-2-(perfuorooctanoyl-N'-methylimino)ethylammonium chloride)-co-(N,N,N-trimethyl-N-4'-vinylbenzylammonium chloride).


**Patentansprüche**

1. Superparamagnetische Eisenoxid-Nanopartikel mit ultradünnen Polymerschichten, **dadurch gekennzeichnet, dass** die Nanopartikel aus:

    a. mit einem Polykation co-präzipitieren superparamagnetischen Eisenoxid-Kernen,
    b. einer hydrophilen Beschichtung aus mindestens einer ultradünnen Schicht eines Polyelektrolyten bestehen.

2. Die superparamagnetische Eisenoxid-Nanopartikel gemäß Anspruch 1, **dadurch gekennzeichnet, dass** die erste ultradünne Schicht des Polyelektrolyten infolge der Co-Präzipitation von Nanopartikeln in der Anwesenheit von Polymer gewonnen wird und die anderen ungeraden Schichten, die auf der Oberfläche der erhaltenen Nanopartikel aufgetragen werden, aus einem Polykation bestehen.

3. Superparamagnetische Eisenoxid-Nanopartikel gemäß Anspruch 2, **dadurch gekennzeichnet, dass** das Polykation aus der Gruppe der natürlichen oder modifizierten Polysaccharide gewählt wird, die positiv geladene Gruppen wie Ammonium, Pyridinium oder Phosphonium enthalten.

4. Superparamagnetische Eisenoxid-Nanopartikel gemäß Anspruch 2 oder 3, **dadurch gekennzeichnet, dass** das Polykation ein mit kationischen Ammoniumgruppen modifiziertes Chitosan ist.

5. Superparamagnetische Eisenoxid-Nanopartikel gemäß Anspruch 1 **dadurch gekennzeichnet, dass** die zweite ultradünne Schicht aus Polyelektrolyt und die weiteren geraden Schichten aus einem Polyanion bestehen.

6. Superparamagnetische Eisenoxid-Nanopartikel gemäß Anspruch 5, **dadurch gekennzeichnet, dass** das Polyanion ein Polymer aus der Gruppe der natürlichen oder modifizierten Polysaccharide ist, die negativ geladene Gruppen wie Sulfonat, Sulfat, Carboxyl oder Phosphat enthalten.

7. Superparamagnetische Eisenoxid-Nanopartikel gemäß Anspruch 5 oder 6, **dadurch gekennzeichnet, dass** das Polyanion ein Polymer in Form des anionischen Chitosanderivats oder der Heparin ist.

8. Superparamagnetische Eisenoxid-Nanopartikel gemäß Anspruch 7, **dadurch gekennzeichnet, dass** das Polymer in Form des anionischen Chitosanderivats ein mit Schwefeltrioxidkomplex modifiziertes Carboxymethylchitosan ist.

9. Superparamagnetische Eisenoxid-Nanopartikel gemäß Anspruch 1, **dadurch gekennzeichnet, dass** die Nanopartikel eine Suspension in wässriger Phase bilden.

10. Superparamagnetische Eisenoxid-Nanopartikel gemäß Anspruch 1, **dadurch gekennzeichnet, dass** die Nanopartikel mit einer solchen Anzahl von Schichten beschichtet sind, dass ihre durchschnittliche Größe 5-200 nm und/oder der Durchmesser des Eisenoxid-Kerns 5-20 nm und/oder die Dicke der Polymerbeschichtung 0,1-100 nm betragen.

11. Superparamagnetische Eisenoxid-Nanopartikel gemäß Anspruch 1, **dadurch gekennzeichnet, dass** die hydrophile Beschichtung funktionalisiert wird, indem eine Fluoreszenzsonde und/oder eine Gadoliniumverbindung und/oder Antikörper und/oder Antikörper-Aptamere und/oder eine Polyelektrolytschicht mit fluorierten Gruppen daran befestigt werden.

12. Superparamagnetische Eisenoxid-Nanopartikel gemäß Anspruch 11, **dadurch gekennzeichnet, dass** die Fluoreszenzsonde aus der Gruppe von Fluorescamin, Fluorescein, Cyanin, Rhodaminen oder funktionalisierten Quantenpunkten gewählt wird.

13. Superparamagnetische Eisenoxid-Nanopartikel gemäß Anspruch 11, **dadurch gekennzeichnet, dass** die Gadoliniumverbindung aus der Gruppe von Chelataten von Gadolinium-Ionen mit Diethylentriaminpentaessigsäure (DTPA) oder Ethylendiamintetraessigsäure (EDTA) gewählt wird.

14. Superparamagnetische Eisenoxid-Nanopartikel gemäß Anspruch 11, **dadurch gekennzeichnet, dass** der Antikörper aus der Gruppe von monoklonalen Antikörpern gegen Zelladhäsionsmoleküle, insbesondere VCAM-1, monoklonalen Antikörpern gegen P-Selektin oder monoklonalen Antikörpern gegen E-Selektin gewählt wird.

15. Superparamagnetische Eisenoxid-Nanopartikel gemäß Anspruch 11, **dadurch gekennzeichnet, dass** die Antikörper-Aptamere aus der Gruppe von monoklonalen Antikörper-Aptameren gegen Zelladhäsionsmoleküle, gegen P-Selektin oder E-Selektin gewählt werden.

16. Superparamagnetische Eisenoxid-Nanopartikel gemäß Anspruch 11, **dadurch gekennzeichnet, dass,** die Polyelektrolytschichten mit fluorierten Gruppen Poly(N,N-Dimethyl-N-4'-vinylbenzylN-2-(perfluorooctanol-N'-methylimino)ethylammoniumchlorid) -Co-(N,N,N,N-Trimethyl-N4'-vinylbenzylammoniumchlorid) sind.

## Revendications

1. Les nanoparticules superparamagnétiques d'oxyde de fer à couches polymères ultra-minces, **caractérisées en ce que** les nanoparticules sont constituées :

   a) de noyaux superparamagnétiques d'oxydes de fer coprécipités avec polycation,
   b) d'une couche hydrophile constituée d'au moins une couche ultra-mince de polyélectrolyte.

2. Les nanoparticules superparamagnétiques d'oxyde de fer selon la revendication 1, **caractérisées en ce que** la première couche ultra-mince de polyélectrolyte est obtenue par coprécipitation des nanoparticules en présence de polymère et que les autres couches impaires appliquées sur la surface des nanoparticules obtenues sont constituées de polycation.

3. Les nanoparticules superparamagnétiques d'oxyde de fer selon la revendication 2, **caractérisées en ce que** le polycation est choisi dans le groupe des polysaccharides naturels ou modifiés contenant des groupes chargés positivement tels que : ions ammonium, pyridine ou phosphonium.

4. Les nanoparticules superparamagnétiques d'oxyde de fer selon la revendication 2 ou 3, **caractérisées en ce que** le polycation est un chitosane modifié avec des groupes de cation ammonium.

5. Les nanoparticules superparamagnétiques d'oxyde de fer selon la revendication 1, **caractérisées en ce que** la deuxième couche ultra-mince de polyélectrolyte et plus de couches paires est constituée de polyanion.

**6.** Les nanoparticules superparamagnétiques d'oxyde de fer selon la revendication 5, **caractérisées en ce que** le polyanion est un polymère du groupe constitué de polysaccharides naturels ou modifiés contenant des groupes fonctionnels chargés négativement tels que groupe sulfone, sulfate, groupe carboxyle ou phosphate.

**7.** Les nanoparticules superparamagnétiques d'oxyde de fer selon la revendication 5 ou 6, **caractérisées en ce que** le polyanion est un polymère dérivé du chitosane anionique ou l'héparine.

**8.** Les nanoparticules superparamagnétiques d'oxyde de fer selon la revendication 7, **caractérisées en ce que** le polymère formant le dérivé du chitosane anionique est le carboxyméthyl du chitosane modifié d'un complexe de trioxyde de soufre.

**9.** Les nanoparticules superparamagnétiques d'oxyde de fer selon la revendication 1, **caractérisées en ce que** les nanoparticules forment une suspension en phase aqueuse.

**10.** Les nanoparticules superparamagnétiques d'oxyde de fer selon la revendication 1, **caractérisées en ce que** les nanoparticules sont revêtues d'un nombre de couches de revêtement tel que leur taille moyenne est de 5 à 200 nm et/ou que le diamètre du noyau d'oxyde de fer est de 5 à 20 nm et/ou que l'épaisseur de la couche de revêtement polymère est de 0,1 à 100 nm.

**11.** Les nanoparticules superparamagnétiques d'oxyde de fer selon la revendication 1, **caractérisées en ce que** la couche de revêtement hydrophile est fonctionnalisée par l'adjonction d'une sonde fluorescente et/ou d'un composé de gadolinium et/ou d'anticorps et/ou d'aptamères d'anticorps et/ou d'une couche polyélectrolyte contenant les groupes fluorés.

**12.** Les nanoparticules superparamagnétiques d'oxyde de fer selon la revendication 11, **caractérisées en ce que** la sonde fluorescente est choisie dans un groupe constitué de fluoreskamine, fluorescéine, cyanine, rhodamine ou points quantiques fonctionnalisés.

**13.** Les nanoparticules superparamagnétiques d'oxyde de fer selon la revendication 11, **caractérisées en ce que** le composé du gadolinium est choisi dans le groupe constitué de ions de chélates de gadolinium avec l'acide diéthylènetriaminopentaacétique (DTPA) ou l'acide éthylène diamine tétraacétique (EDTA).

**14.** Les nanoparticules superparamagnétiques d'oxyde de fer selon la revendication 11, **caractérisées en ce que** l'anticorps est choisi dans un groupe composé d'anticorps monoclonaux bloquant les molécules d'adhésion cellulaire, notamment VCAM-1, d'anticorps monoclonaux bloquant la P-selectine ou d'anticorps monoclonaux bloquant la E-selectine.

**15.** Les nanoparticules superparamagnétiques d'oxyde de fer selon la revendication 11, **caractérisées en ce que** les aptamères d'anticorps sont choisis dans un groupe composé d'aptamères d'anticorps monoclonaux bloquant les molécules d'adhésion cellulaire, la sélectine P ou E.

**16.** Les nanoparticules superparamagnétiques d'oxyde de fer selon la revendication 11, **caractérisées en ce que** les couches de polyélectrolyte contenant des groupes fluorés sont le poly(chlorite N,N-diméthyl-N-4'-vinylbenzyl-N-2-(perfluorooctane-N'-méthylolamine)éthylamine)-co-(chlorite N,N,N-triméthyl-N-4'-vinylbenzylammonium)

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 9962079 A
- US 2004146855 A1
- US 5262176 A

## Non-patent literature cited in the description

- **AGNIHOTRI, S.A. ; MALLIKARJUNA, N.N. ; AMINABHAVI, T.M.** *Journal of Controlled Release,* 2004, vol. 100, 5-28
- **BULWAN, M. ; ZAPOTOCZNY, S. ; NOWAKOWSKA, M.** *Soft Matter,* 2009, vol. 5, 4726-4732
- **CHO, J. ; GRANT, J. ; PIQUETTE-MILLER, M. ; ALLEN, C.** *Biomacromolecules,* 2006, vol. 7
- **GERALDES, C.F.G.C. S. ; LAURENT, C.F.G.C.** *Contrast Media Mol. Imaging,* 2009, vol. 4, 1-23
- **GUPTA, A.K. ; GUPTA, M.** *Biomaterials,* 2005, vol. 26, 3995-4021
- **HERNÁNDEZ, R. ; ZAMORA-MORA, V. ; SIPAJA-BALLESTERO, M. ; VEGA-BAUDRIT, J. ; LOPEZ, D. ; MIJANGOS, C.** *Journal of Colloid and Interface Science,* 2009, vol. 339, 53-59
- **IIDAA, H. ; TAKAYANAGIA, K. ; NAKANISHIB, T. ; OSAKAA, T.** *Journal of Colloid and Interface Science,* 2007, vol. 314, 274-280
- **JUNG, C.W. ; JACOBS, P.** *Magnetic Resonance Imaging,* 1995, vol. 13, 661-674
- **KWAK, S. ; LAFLEUR, M.** *Macromolecules,* 2003, vol. 36, 3189-3195
- **LAURENT, S. ; FORGE, D. ; PORT, M. ; ROCH, A. ; ROBIC, C. ; VANDER ELST, L. ; MULLER, R.N.** *Chemical Reviews,* 2008, vol. 108, 2064-2110
- **TARTAJ, P. ; PUERTO MORALES, M. ; VEINTEMILLAS-VERDAGUER, S. ; GONZÁLEZ-CARREÑO, T. ; SERNA, C.J.** *Journal of Physics D: Applied Physics,* 2003, vol. 36, R182-R197
- **TARTAJ, P. ; MORALES, M.P. ; GONZÁLEZ-CARREÑO, T. ; VEINTEMILLAS-VERDAGUER, S. ; SERNA, C.J.** *Journal of Magnetism and Magnetic Materials,* 2005, vol. 290-29, 28-34
- **TEJA, A.S. ; PEI-YOONG KOH.** *Progress in Crystal Growth and Characterization of Materials,* 2009, vol. 55, 22-45
- **WANG, Y. ; LI, B. ; ZHOU, Y ; JIA, D.** *Advanced Technologies,* 2008, vol. 19, 1256-1261
- **YUAN, Q ; VENKATASUBRAMANIAN, R. ; HEIN, S. ; MISRA, R.D.K.** *ActaBiomaterialia,* 2008, vol. 4, 1024-1037
- **GERALDES, C.F.G.C. S ; LAURENT, C.F.G.C.** *Contrast Media Mol. Imaging,* 2009, vol. 4, 1-23
- **HERNÁNDEZ, R. ; ZAMORA-MORA, V. ; SIPAJA-BALLESTERO, M. ; VEGA-BAUDRIT, J. ; LÓPEZ, D. ; MIJANGOS, C.** *Journal of Colloid and Interface Science,* 2009, vol. 339, 53-59
- **LAURENT, S ; FORGE, D. ; PORT, M. ; ROCH, A. ; ROBIC, C. ; VANDER ELST, L. ; MULLER, R.N.** *Chemical Reviews,* 2008, vol. 108, 2064-2110
- **TEJA, A.S. ; PEI-YOONG KO.** *Progress in Crystal Growth and Characterization of Materials,* 2009, vol. 55, 22-45
- **WANG, Y. ; LI, B. ; ZHOU, Y. ; JIA, D.** *Polymers for Advanced Technologies,* 2008, vol. 19, 1256-1261
- **YUAN, Q. ; VENKATASUBRAMANIAN, R. ; HEIN, S. ; MISRA, R.D.K.** *ActaBiomaterialia,* 2008, vol. 4, 1024-1037